# EUROPEAN PATENT SPECIFICATION

(11) **EP 0 891 764 B1**
(45) Date of publication and mention of the grant of the patent: **18.02.2004**
(21) Application number: 98113111.3
(22) Date of filing: 14.07.1998
(51) Int. Cl.: A61K 7/06, A61K 7/48

(54) **Ethanolic solutions of N-pentadecanoylisoleucine and use thereof in cosmetics**
Ethanolische Lösungen von N-Pentadecanoylisoleucin und Verwendung derselben in der Kosmetik
Solutions de N-pentadecanoylisoleucine et leur utilisation en cosmétique

(30) Priority: 14.07.1997 JP 18809097
(43) Date of publication of application: 20.01.1999
(73) Proprietor: KASHIMA OIL COMPANY, Chiyoda-ku, Tokyo 102 (JP)
(72) Inventor: Ishigami, Kazunori, Tokyo (JP); Takeda, Mitsunori, Tokyo (JP); Ogihara, Kimihiko, Tokyo (JP); Ishizuka, Tatsushi, Tokyo (JP)
(74) Representative: VOSSIUS & PARTNER

(56) References cited:
- EP-A- 0 839 515
- CHEMICAL ABSTRACTS, vol. 126, no. 12, 24 March 1997 Columbus, Ohio, US; abstract no. 157809, ISHIGAMI KAZUNORI E.A.: "Preparation of N-acylamino acids as surfactants having disinfectant, metal-corrosion inhibiting, hair growth-promoting and moisturizing activities" XP002081552 & JP 08 337563 A (KASHIMA SEKYU)
- DATABASE WPI Derwent Publications Ltd., London, GB; AN 97103674 XP002081553 OGIWARA KIMIHIKO: "N-acyamino acid composition used in e.g. detergents-pepd. by condensing higher fatty acid chloride and amino acid" & JP 08 337562 A (KASHIMA SEKIYU) , 24 December 1996

## Description

The present invention relates to cosmetics such as a hair growth agent and a hair grooming agent. More particularly, the present invention is concerned with cosmetics which have excellent hair growth-promoting effect, humectant action, subcutaneous blood flow rate-increasing action and which are well suited for hair growth, skin care and also with N-pentadecanoylisoleucine as an effective ingredient of said cosmetics which has markedly excellent solubility in ethanol at the time of preparing a pharmaceutical preparation of the above-stated cosmetics.

Cosmetics blended with various effective drugs have heretofore been known. For example, a hair growth agent is incorporated with, as an effective drug, a vitamin such as vitamin E; an amino acid such as serine and methionine; a vasodilator such as an acetylcholine derivative; an antiflammatory agent such as lithospermum root extract; a female sex hormone such as estradiol ; a skin function enhancing agent such as cepharanthine; a melanin synthesis catalyst such as copper pantothenate; a keratolytics such as salicylic acid; and the like, said hair growth agent being used for the prevention and therapy of alopecia.

As examples in which a fatty acid or a derivative thereof is blended in cosmetics such as a hair growth agent, some products are known in which a natural vegetable oil, such as olive oil or castor oil, or stearic acid is blended for the purpose of improving the physical properties. It is known that almost all fatty acids which constitute various lipids of natural origin, such as vegetable oils and animal oils, are fatty acids havingan even-numbered carbon atoms chain, whether the fatty acids are saturated fatty acids such as stearic acid, palmitic acid or the like, or unsaturated fatty acids such as oleic acid, linolenic acid or the like.

On the other hand, examples in which fatty acids having odd-numbered carbon atoms or derivatives thereof are incorporated in hair cosmetics include the compounds described, for example, in Japanese Patent Publication No. 41363/1988( Sho-63 ) and Japanese Patent Application Laid - Open No. 337515 /1996 (Hei-8).

JP-08 337563 A discloses N-acylamino acids having an acyl group which consists of 13 to 17 odd carbon atoms and salts thereof. The compound having surface active, bactericidal, metal corrosion inhibiting, trichogenic, humectant, and subcutaneous blood flow increasing actions is useful as a hair tonic, detergent, dispersing agent, emulsifier, antibacterial agent and antiseptic agent.

JP-08 337562 A discloses a composition comprising particular N-acylamino acids having an odd carbon number acyl group or a salt thereof. The N-acylamino acid constitutes the main component in the composition useful as a detergent, dispersing agent, emulsifier, antibacterial agent, antiseptic agent and ultraviolet absorber.

It is said that the aforesaid conventional cosmetics such as the hair growth agent, especially the cosmetics described in the above-mentioned Japanese Patent Application Laid-Open No. 337515 /1996( Hei-8 ) ) are effective for the prevention and improvement of dandruff, itch, hair falling out and the like, and further promote hair generation and restoring. However, the N-acylamino acids as the effective ingredient in said cosmetics, when being dissolved in a solvent such as ethanol in order to prepare the cosmetics, are not sufficient in their solubility with the result that the necessary performance of the hair growth agent, etc. is impaired. In view of the foregoing, it has eagerly been desired that said N-acylamino acids be improved in their solubility, particularly at an low temperature.

The present invention has been contrived under such circumstances. Thus, the object of the present invention is to provide cosmetics that are excellent in hair growth - promoting effect, humectant action and subcutaneous blood flow rate-increasing action, and thus are well suited to use for hair growth and skin care.

As a result of intensive research and investigation accumulated by the present inventors in order to achieve the above-mentioned object, it has been found that specific acylamino acids having a pentadecanoyl group or salts thereof are remarkably excellent in the foregoing solubility, and that excellent effect is exhibited by the use thereof as an effective ingredient, in regard to hair growth-promoting effect, humectant action and subcutaneous blood flow rate-increasing action. The present invention has been accomplished by the above-mentioned findings and information.

Specifically, the present invention provides a cosmetic comprising N-pentadecanoylisoleucine or a salt thereof and ethanol.

The salt of said N-pentadecanoylisoleucine is exemplified by salts of alkali metals such as sodium and potassium; salts of alkaline earth metals such as calcium; and salts of ethanolamines such as monoethanolamine, diethanolamine and triethanolamine.

The blending amount of said N-pentadecanoylisoleucine or the salt thereof in the cosmetic of the invention can appropriately be determined according to the purpose of use and the mode of formulation, and the blending amount thereof is usually selected from the range of 0.01 to 10% by weight based on the whole amount of the cosmetic. The blending amount thereof, when less than 0.01% by weight, often leads to the failure of the exertion of the working effect of the invention. On the contrary, the blending amount, when more than 10% by weight, is not favorable from the viewpoint of the economical efficiency, since the working effect thereof is not enhanced in proportion to the blended amount.

Since said N-pentadecanoylisoleucine can exert expected effects in the above blending amounts and further has an excellent solubility in ethanol, the solution stability is enhanced especially at low temperatures, when made into preparations, enabling to reduce the amount of additives,such as solubilizers and dispersants, to be added for enhancing the solubility.

The aforesaid N-acylamino acids can be synthesized, for example, by the condensation reaction between higher fatty acid chlorides having odd-numbered carbon atoms and amino acids in accordance with so called Schotten-Baumann reaction or the process described in "Studies on lipoamino acids (I )", Seikagaku, vol.35, No.2, pp 67 to 74, 1963. The higher fatty acids having odd-numbered carbon atoms can be synthesized by a conventional method, specifically by the oxo process from α-olefins having even-numbered carbon atoms and by the process using microorganism as described in Japanese Patent Application Laid-Open No.253866/1994(Hei-6).

The cosmetic according to the present invention can be prepared from the aforestated N-pentadecanoylisoleucine or a salt thereof by the use of ethanol.

The cosmetic according to the present invention may be blended, at need, with other components to the extent that such blending does not impair the object of the present invention, in addition to the principal ingredient such as the aforestated N-pentadecanoylisoleucine or the salt thereof. Such other components are appropriately selected in accordance with the purpose of use, type and form ot the cosmetic, and are exemplified by base materials such as distilled water, alcohols, polyhydric alcohols, surfactants, fats and oils, and polysaccharides, colorants, perfumes, vitamins, amino acids, hormones, vasodilators, antiflammatory agents, keratolytics, disinfectants, bactericides, fungicides and antiseptics. In addition, the cosmetic according to the present invention can be prepared into a variety of forms such as liquid, powder and paste, and can be made into various marketable products such as a hair growth agent and a hair grooming agent.

According to the present invention, it is made possible to obtain a cosmetic that is well suited for the use as hair growth and skin care agents and also for promoting hair growth by the use of a composition comprising N-pentadecanoylisoleucine or a salt thereof.

In the following, the present invention will be described in more detail with reference to a comparative example and working examples.

### Preparation Example 1

### (1) Preparation of pentadecanoyl chloride

In a one ℓ two-necked flask was placed 430.4 g (1.78 mol) of pentadecanoic acid, and the flask was equipped with a 200 mℓ dropping funnel containing 258.1 g (2.17 mol) thionyl chloride and a reflux condenser. Then the pentadecanoic acid was completely dissolved under stirring and heating of the flask in an oil bath at 90°C. Thereafter the thionyl chloride in the dropping funnel was added dropwise to the pentadecanoic acid in the flask over a period of 2 hours, and further the oil bath was maintained at 90°C for one hour . The unreacted thionyl chloride was removed from the resultant reaction mixture under reduced pressure by means of an aspirator. The crude reaction product thus obtained was subjected to vacuum distillation. Thus 429.7 g (1.65 mol) of pentadecanoyl chloride was obtained in a yield of 93% based on the fed pentadecanoic acid.

### (2) Synthesis of N-pentadecanoyl-L-isoleucine

In a one ℓ three-necked flask were placed 40g ( 0.30 mol ) of L-isoleucine and an aqueous solution of 12.0g ( 0.30 mol ) of sodium hydroxide in 600 mℓ of water under stirring. After the L-isoleucine was dissolved, 400 mℓ of acetone was added to the mixture in the flask. Then, to the resultant reaction liquid which was cooled to 0°C were added dropwise over a period of one hour, 79.5 ( 0.30 mol ) of pentadecanoyl chloride and simultaneously, an aqueous solution of 12.2g ( 0.30 mol ) of sodium hydroxide in 120 mℓ of water, while maintaining the reaction temperature at 0°C and pH at 11 to 12. Then, by maintaining the reaction temperature at 0°C under stirring over a period of one hour, a solution of N-pentadecanoyl-L-isoleucine sodium salt was obtained. To the resultant solution was added 61 mℓ of 5 N hydrochloric acid to lower the pH of the solution to 1 and precipitate crystal . The precipitated crystal was separated from the solution by means of vacuum filtration, washed with water and dried under reduced pressure. The resultant crystal was washed with 600 mℓ of hexane, dried and recrystallized from a mixed solvent of ethanol/water( 8:2 ). As a result, 63.8g ( 0.18 mol ) of objective N-pentadecanoyl-L-isoleucine according to the invention were obtained in a yield of 60% based on the fed pentadecanoyl chloride.

### Example 1 and Comparative Examples 1 to 7

A group of eight male mice aged 8 weeks of C3H series with a body weight of 18 to 24g was subjected to depilation at a back portion thereof in a size of about 2× 3.5 cm. Then, each of the N-acylamino acid compounds as shown in Table 1 was dissolved in ethanol to a concentration of 1% by weight as a sample to be tested. Then 0.1 mℓ of the sample were applied to the depilated back portions of the mice once a day over a period of 3 weeks to visually observe the hair growth state. An evaluation was made of the hair growth effect by visually observing the state of trichogenous promotion in comparison with the back portions coated only with ethanol on the basis of the criterion in Table 1. The results are given in Table 1 . With regard to general sympton, dermal state and change in body weight, no influence by the applying of any of the samples tested was recognized throughout the testing period.In addition, an investigation was made of the solubilities of the foregoing N-acylamino acid compounds in 70% ethanol at 10°C. The results are given also in Table 1. The N-acylamino acid compounds that were used in this test as Comparative Examples had been synthesized in accordance with the Preparation Example 1.

**Table 1**

| | N-acylamino acid | Retrichogenous effect | Solubility (w/w %) |
|---|---|---|---|
| Example 1 | N-PDNY-L-isoleucine | ⓞ | 4.0 |
| Comp. Example 1 | N-PDNY-L-alanine | ⓞ | 0.8 |
| Comp. Example 2 | N-PDNY-L-leucine | ⓞ | 2.1 |
| Comp. Example 3 | N-PDNY-L-methionine | ⓞ | 3.8 |
| Comp. Example 4 | N-PDNY-L-valine | ⓞ | 2.5 |
| Comp. Example 5 | N-PDNY-L-phenyl-alanine | ⓞ | 3.7 |
| Comp. Example 6 | N-PDNY-L-tyrosine | ⓞ | 0.7 |
| Comp. Example 7 | N-PDNY-L-asparatic acid | ○ | 0.3 |
| [Remarks] PDNY: pentadecanoyl ⓞ : rapid and excellent retrichogenous action ○ : rapid and good retrichogenous action Δ : slow and slight retrichogenous action | | | |

In the following, a variety of cosmetics along with forms and chemical compositions are given as additional examples according to the present invention.

### Comp. Example 8 (Hair grooming agent)

| | % by weight |
|---|---|
| Ethanol | 78.0 |
| N-pentadecanoylalanine | 0.5 |
| Olive oil | 1.0 |
| α-Tocopherol | 0.5 |
| Perfume | 0.5 |
| Purified water | 19.5 |
| Antiseptics | (proper amount) |

### Comp. Example 9 (Hair grooming agent)

| | % by weight |
|---|---|
| Ethanol | 60.0 |
| N-pentadecanoylleucine | 0.5 |
| Dipropylene glycol | 3.0 |
| Perfume | 0.5 |
| Purified water | 36.0 |
| Antiseptics | (proper amount) |

### Comp. Example 10 (Hair liquid)

| | % by weight |
|---|---|
| Ethanol | 40.0 |
| N-pentadecanoylmethionine | 2.0 |
| Glycerol | 1.0 |
| Polyoxypropylene(40mol) butyl ether | 10.0 |
| Perfume | 0.5 |
| Purified water | 46.5 |
| Antiseptics | (proper amount ) |

### Comp. Example 11 (Hair grooming agent)

| | % by weight |
|---|---|
| Ethanol | 78.0 |
| N-pentadecanoylvaline | 0.5 |
| Olive oil | 1.0 |
| α-Tocopherol | 0.5 |
| Perfume | 0.5 |
| Purified water | 19.5 |
| Antiseptics | (proper amount) |

### Example 2 (Hair grooming agent)

| | % by weight |
|---|---|
| Ethanol | 60.0 |
| N-pentadecanoylisoleucine | 0.5 |
| Dipropylene glycol | 3.0 |
| Perfume | 0.5 |
| Purified water | 36.0 |
| Antiseptics | (proper amount) |

### Comp. Example 12 (Hair liquid)

| | % by weight |
|---|---|
| Ethanol | 40.0 |
| N-pentadecanoylphenylalanine | 2.5 |
| Glycerol | 1.0 |
| Polyoxypropylene(40mol) butyl ether | 10.0 |
| Perfume | 0.5 |
| Purified water | 46.0 |
| Antiseptics | (proper amount ) |

## Claims

1. Cosmetic comprising N-pentadecanoylisoleucine or a salt thereof and ethanol.

2. The cosmetic according to Claim 1, wherein said salt is a salt of any of alkali metals, alkaline earth metals and ethanolamines.

3. The cosmetic according to Claim 1, wherein the blending amount of said N-pentadecanoylisoleucine or the salt thereof is in the range of 0.01 to 10 % by weight based on the whole amount of said cosmetic.

4. Use of a composition comprising N-pentadecanoylisoleucine or a salt thereof and ethanol for promoting hair growth.

## Patentansprüche

1. Kosmetikum umfassend N-Pentadecanoylisoleucin oder ein Salz davon und Ethanol.

2. Kosmetikum gemäß Anspruch 1, wobei das Salz ein Salz von Alkalimetallen, Erdalkalimetallen oder Ethanolaminen ist.

3. Kosmetikum gemäß Anspruch 1, wobei die Mischungsmenge des N-Pentadecanoylisoleucins oder des Salzes davon im Bereich von 0,01 bis 10 Gew.-%, bezogen auf die Gesamtmenge des Kosmetikums, liegt.

4. Verwendung einer Zusammensetzung umfassend N-Pentadecanoylisoleucin oder ein Salz davon und Ethanol zur Förderung von Haarwachstum.

## Revendications

1. Produit cosmétique comprenant de la N-pentadécanoylisoleucine ou un sel de celle-ci et de l'éthanol.

2. Produit cosmétique selon la revendication 1, dans lequel ledit sel est un sel de métaux alcalins, de métaux alcalino-terreux ou d'éthanolamines.

3. Produit cosmétique selon la revendication 1, dans lequel la quantité en mélange de ladite N-pentadécanoylisoleucine ou du sel de celle-ci est dans la plage de 0,01 à 10 % en poids sur la base de la quantité totale dudit produit cosmétique.

4. Utilisation d'une composition comprenant de la N-pentadécanoylisoleucine ou un sel de celle-ci et de l'éthanol pour activer la croissance des cheveux.
